# EUROPEAN PATENT SPECIFICATION

(11) **EP 3 351 535 B1**
(45) Date of publication and mention of the grant of the patent: **28.08.2019**
(21) Application number: 17001787.5
(22) Date of filing: 31.10.2017
(51) Int. Cl.: C07D 233/72, C07F 11/00

(54) **PROCESS FOR THE PREPARATION OF 5-SELENOCYANATO-URACIL**
VERFAHREN ZUR HERSTELLUNG VON 5-SELENOCYANATO-URACIL
PROCÉDÉ DE LA PRÉPARATION DE 5-SELENOCYANATO-URACIL

(30) Priority: 31.10.2016 PL 41932316
(43) Date of publication of application: 25.07.2018
(73) Proprietor: Uniwersytet Gdanski, 80-309 Gdansk (PL)
(72) Inventor: Sosnowska, Marta, 83-000 Pruszcz Gdanski (PL); Makurat, Samanta, 80-462 Gdansk (PL); Chomicz-Manka, Lidia, 80-515 Gdansk (PL); Rak, Janusz, 80-353 GDANSK (PL)
(74) Representative: Pawlowska, Justyna

(56) References cited:
- WO-A1-2006/063408
- MARTA SOSNOWSKA ET AL: "5-Selenocyanatouracil: A Potential Hypoxic Radiosensitizer. Electron Attachment Induced Formation of Selenium Centered Radical", JOURNAL OF PHYSICAL CHEMISTRY PART B: CONDENSED MATTER, MATERIALS, SURFACES, INTERFACES & BIOPHYSICAL, vol. 121, no. 25, 15 June 2017 (2017-06-15), pages 6139-6147, XP055466320, US ISSN: 1520-6106, DOI: 10.1021/acs.jpcb.7b03633
- SAMANTA MAKURAT ET AL: "Electrophilic 5-Substituted Uracils as Potential Radiosensitizers: A Density Functional Theory Study", CHEMPHYSCHEM - A EUROPEAN JOURNAL OF CHEMICAL PHYSICS & PHYSICALCHEMISTRY., vol. 17, no. 16, 18 August 2016 (2016-08-18), pages 2572-2578, XP055466275, DE ISSN: 1439-4235, DOI: 10.1002/cphc.201600240
- CRAIG M. ROBERTSON ET AL: "Enhanced Conductivity and Magnetic Ordering in Isostructural Heavy Atom Radicals", JOURNAL OF THE AMERICAN CHEMICAL SOCIETY, vol. 130, no. 26, 1 July 2008 (2008-07-01) , pages 8414-8425, XP055466284, ISSN: 0002-7863, DOI: 10.1021/ja801070d

## Description

The object of the invention is a new method of synthesis of the uracil derivative (5-selenocyanatouracil), possessing a radiosensitizing potential.

Radiotherapy (RT), similarly to chemotherapy is one of the most popular cancer treatment methods that is being applied to about 50% of all cancer patients. The method employs ionizing radiation (IR), mainly via the indirect pathway - using water radiolysis products (hydroxyl radical, particularly), which are prone to damage cellular DNA. Although it is being widely used, RT is associated with a number of side effects and its power is additionally diminished by the main characteristic of many solid tumors - hypoxia. This feature makes the cancer cells up to three times more radioresistant than the surrounding tissue. The lowered oxygen level in cancer cells requires higher therapeutic doses of RT since most of hydroxyl radicals (responsible for the DNA damage) are useless in hypoxic conditions.

To increase efficiency of DNA damage caused by RT, radiosensitizers that work at the lowered oxygen levels environment could be applied. One of the group of such radiosensitizers are nucleobases, prone to incorporate into the cellular DNA during its biosynthesis. The mechanism of radiosensitization employs solvated electrons formed during water radiolysis process under hypoxic conditions. The subtle modification of pyrimidine base, like substituting the C5 position hydrogen by halogen atom in uracil (U), changes its electron acceptor properties and allows the modified U to convert into the 5-uracilyl radical (dU•) after solvated electron attachment. This highly reactive dU• radical is further able to damage DNA and, as a consequence - leads to cancer cell's death. The radiosensitizing properties of 5-iodo- and 5-bromouracil were thoroughly studied in the past, both *in vitro* and *in vivo,* but despite promising *in vitro* results, the clinical trials showed insufficient profit of their use, and therefore, prevented introducing them into clinical practice. Therefore it is highly justified to search for new compounds (radiosensitizers, that would be able to work in hypoxic environment), that could be further used in radiotherapy combined cancer treatment.

5-Selenocyanatouracil (U-SeCN) seems to be the promising uracil derivative possesing the described above properties. This compound was proposed for the first time in "Electrophilic 5-Substituted Uracils as Potential Radiosensitizers: A Density Functional Theory Study" by S. Makurat, L. Chomicz-Mańka, J. Rak (ChemPhysChem, DOI: 10.1002/cphc.201600240; see attachment no. 1). In that paper a few modified uracils were proposed, and their radiosensitizing potential was investigated with the use of computational chemistry methods. The Density Functional Theory (DFT) methods used therein allowed for the determination of dissociative electron attachment (DEA) free energy profiles for a set of molecules, including U-SeCN. The theoretical model foresees that after initial electron attachment, the radical anion [U-SeCN]•- is formed and immediately undergoes a barrier-free dissociation, leading to the cytotoxic CN-anion release and U-Se• radical formation. Moreover, the theromodynamic stimuli (ΔG) for the U-SeCN DEA processs is about -80.0 kcal/mol (15 kcal/mol more than ΔG obtained for the analogical DEA process of 5-bromouracil - a well-known radiosensitizer,).

The compound, being the subject of the invention shown here, is a potential radiosensitizer of ionizing radiation induced DNA damage process, that could be used during radiotherapy employed in cancer treatment. The derivative proposed here, if efficiently incorporated into cellular DNA, should enhance its degradation in hypoxic conditions. That is due to the attachemnt of solvated electrons (produced during water radiolysis), which are inactive toward the native, unmodified DNA. According to our knowledge, this type of radiosensitizers have not been used in the radiotherapy so far. Moreover, due to the mechanism of the sensitizer action, involving incorporation of the U-SeCN compound (uracil derivative that is a subject of this application) to the cell genome with the use of replocation machinery, the solution proposed here appears to be a universal method supporting the solid tumors radiotherapy (as cancer cells usually undergo more intensive divisions, using the same biochemical machinery to make copies of its own genetic material).

The new method of 5-selenocyanatouracil (U-SeCN) synthesis was developed. The compound was then purified and MS, NMR, IR spectra were recorded in order to identify the product. The aqueous solution containing synthesized U-SeCN was then subjected to X irradiation in reductive conditions and, following HPCL analysis, proved to undergo DEA process induced by solvated electrons formed during water radiolysis.

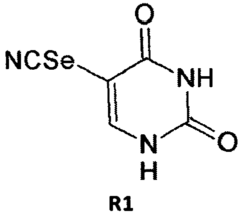

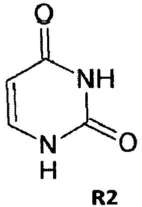

KSeCN **R3**

CISeCN **R4**

The object of the present invention is a method of synthesis of the uracil derivative ((5-selenocyanatouracil, molecular formula R1), which is obtained in the following steps:
- in anhydrous conditions, compound R2 reacts with R4, which is the donor of the selenocyanato (SeCN) group,
- compound R2 reacts with selenocyanogen chloride (molecular formula R4), which has been obtained by the reaction of compound R3:KSeCN with chlorine gas.

The method of synthesis, where compound R2 reacts with compound R4 in a molar ratio of 1:9.

The method of synthesis, where compound R4 is obtained by adding 0.54 g (7.6 mmol) of dry gaseous chlorine and 1.5 g (10.4 mmol) of dried potassium selenocyanate (KSeCN, R3), solvated in a small amount of acetic acid (2-3mL) to 150 mL of acetic acid; after an 1.5 hour 129 mg (1.15 mmol) of uracil is added to the produced selenocyanogen chloride (CISeCN, R4); after 20 hours of the reaction the resulting brown suspension is filtered, and the solvent is evaporated in vacuo.

The method of synthesis, where the method is characterized by separate preparation of a selenocyanating reagent R4 and subsequent addition of compound R2 to it.

### Figures description:

**Fig. 1****.** Chromatogram of the crude product of U-SeCN formation reaction.
**Fig. *2*****.** TOF (-) Mass Spectrum of the crude U-SeCN product.
**Fig. 3****.** Chromatogram of purified U-SeCN.
**Fig. 4****.** TOF (-) Mass Spectrum of purified U-SeCN.
**Fig. 5** MSMS TOF (-) Mass Spectrum for the m/z 215,0198 ion of purified U-SeCN.
**Fig. 6****.** Chromatogram showing U-SeCN (10⁻⁴ M) in 0,03 M *tert*-butanol solution before the irradiation.
**Fig. 7****.** Chromatogram showing U-SeCN (10⁻⁴ M) in 0,03 M *tert*-butanol solution after the irradiation (140 Gy).
**Fig. 8****.** LC-MS analysis of U-SeCN (10⁻⁴ M) in 0,03 M tert-butanol solution after irradiation.
**Fig. 9****.** Chromatogram showing U-SeCN (10⁻⁴ M) w 0,03 M tert-butanol solution after 50°C temperature incubation (40 min).
**Fig. 10****.** Chromatogram showing U-SeCN (10⁻³ M) after 17 h (RT) incubation in 0,1 % solution of formic acid, pH 3.
**Fig. 11****.** Chromatogram showing U-SeCN (10⁻³ M) after 17 h (RT) incubation in 100 mM ammonium acetate solution, pH 5.
**Fig. 12****.** Chromatogram showing U-SeCN (10⁻³ M) after 17 h (RT) incubation in 100 mM ammonium acetate solution, pH 7.
**Fig. 13****.** ¹H NMR spectra (500 MHz, DMSO-d₆, 298 K) registered for U-SeCN derivative.
**Fig. 14****.** ¹³C NMR spectra (500 MHz, DMSO-d₆, 298 K) registered for U-SeCN derivative.
**Fig. 15****.** IR spectra registered for U-SeCN derivative.

The invention was shown at the example presented below, that does not limit its scope.

### Example:

0.54 g (7.6 mmol) of dried, gaseous chlorine and 1.5 g (10.4 mmol) od dried potassium selenocyanate (KSeCN), that was previously solvated in a small amount (2-3 mL) of acetic acid, are introduced into 150 mL of acetic acid. Ater an 1,5 h, 129 mg (1.15 mmol) of uracil is added to the selenocyanogen chloride (CISeCN) formed there. After 20 h of stirring in the room temperature, the resulting suspension is filtered, and the solvent is evaporated in vacuo.

This direct selevtive selenocyanation of uracil (R2) was not described before.

The derivative of a molecular formula R1 can be obtained with a high yield by the reaction of compound R2 and R4 in anhydrous conditions. The R4 compound is introducing the selenocyanate group (SeCN) to R2 in this reaction. In the synthesis method described herein, the compound R2 undergoes the reaction with selenocyanogen chloride shown as R4, obtained from potassium selenocyanate compound, R3 and gas chlroine. Exceptional benefits of this method come from separate preparation of selenocyanating agent, and a subsequent R2 addition to it. In the R1 synthesis method described here, the compound R2 reacts with selenocyanogen chloride in a molar ratio of 1:9.

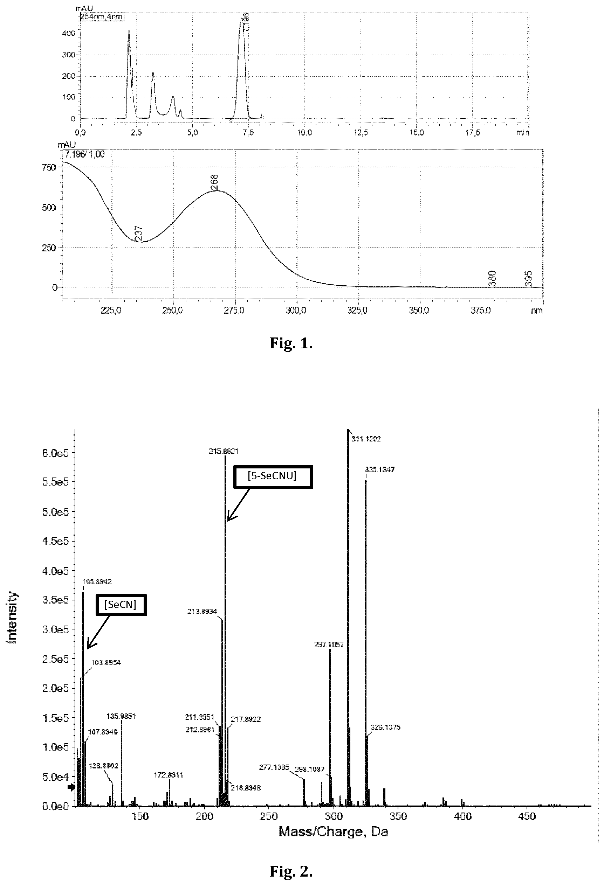

The purification of the crude product was performed with the use of:
(1) High performance liquid chromatograph LC 20AD equipped with a UV SPD M20A (Shimadzu) detector and semi-preparative Phenomenex Synergi Polar-RP reverse-phase column (250 x 10.00 mm dimensions, 4 µm particle size, 100 Å pore size). The mobile phase was formed as a linear gradient of solvents A and B, where: A - 5 mM ammonium acetate, pH 5.5; B - 80% acetonitrile.
A flow rate of 5 mL/min was used and the detector was set to 254 nm to monitor the effluents (Fig.1).
The product was identified with the use of mass spectrometry. The high resolution mass spectrum was obtained via using a TripleTOF 5600 (DuoSpray Ion Source) Sciex instrument working both in positive and negative ionization.

The MS analysis conditions: CUR: 25; GS1: 30; GS2: 30; ISIF: 4500(-); TEM: 300; CE: 10.00; DP: 100. (Fig.2).

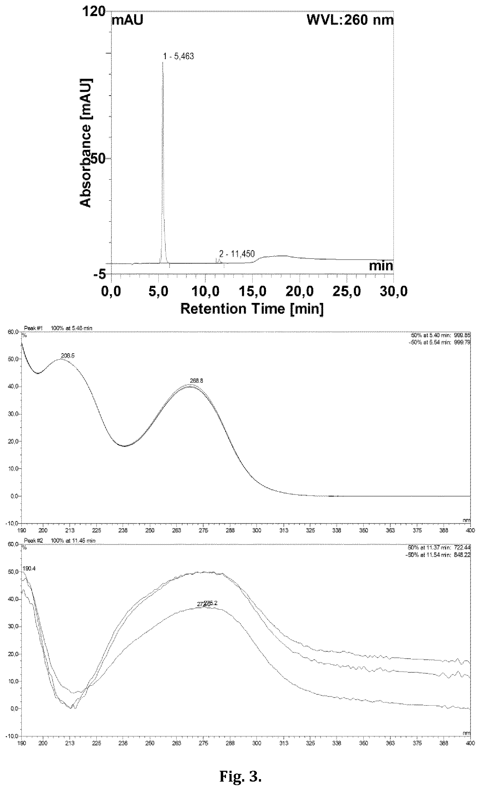

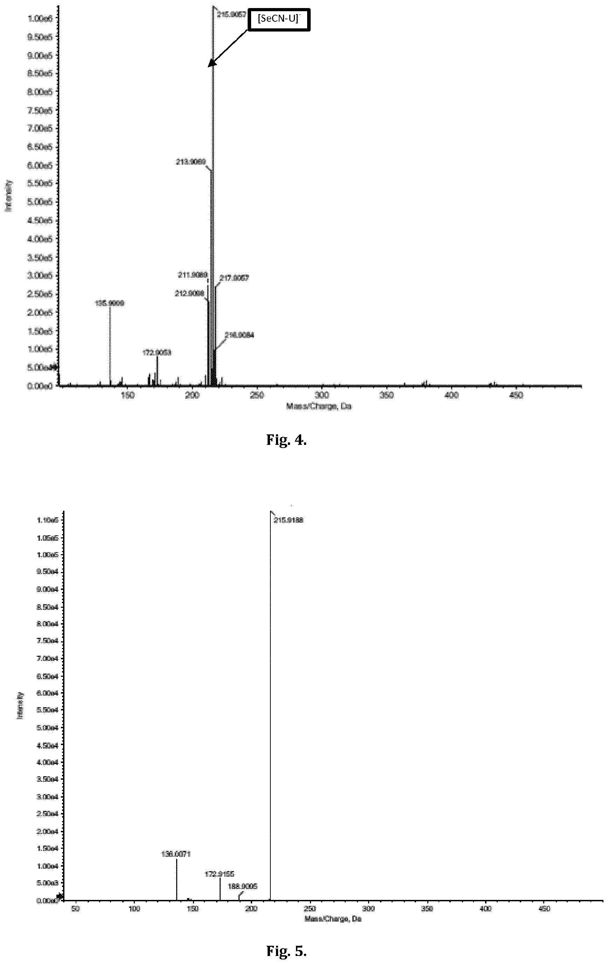

The purity of the product was determined with the use of:
(1) High performance liquid chromatography in reverse phase RP-HPLC, with Dionex Ultimate 3000 (Thermo Scientific) chromatograph and the analityc collumn Wakopak Handy ODS (150 x 4,6 mm). The mobile phase consisted of A' i B' solvents, used in the 30 minutes gradient 0-30% B', where: A' - 0,1 % HCOOH; B' - 80% CH₃CN + 0,1 % HCOOH. The volumetric flow of effluents was equal to 1 mL/min. The detection was set for 260 nm (Fig.3);
(2) High resolution mass spectrometer TripleTOF 5600 (SCIEX). MS analysis conditions: CUR: 25; GS1: 30; GS2: 30; ISIF: 4500(-); TEM: 300; CE: 10.00; DP: 100. (Fig.4, Fig. 5).

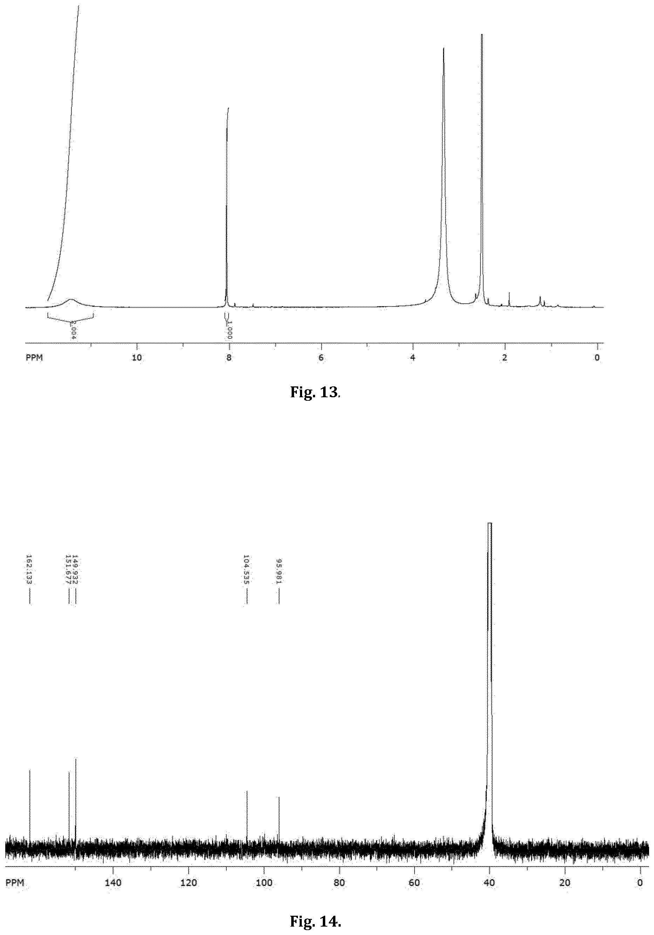

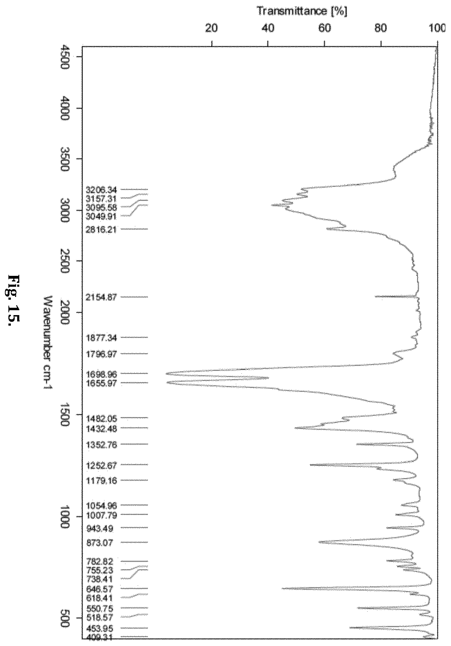

The synthesized compound was analysed with the use of following methods:
(1) IR spectroscopy (IR) - registered in the KBr pellet (Burker IFS66 apparatus) (Tab. 2 and Fig. 15);
(2) magnetic resonance spectroscopy ¹H, ¹³C NMR (Bruker AVANCE III 500 MHz) (Tab.2, Fig. 13 and 14).
Additionally, the molar absorption coefficient was determined (Perkin Elmer Lambda 45 spectrometer) (Tab.2).

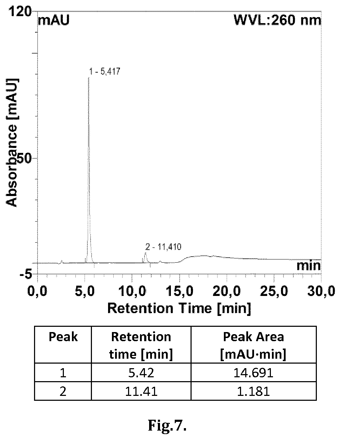

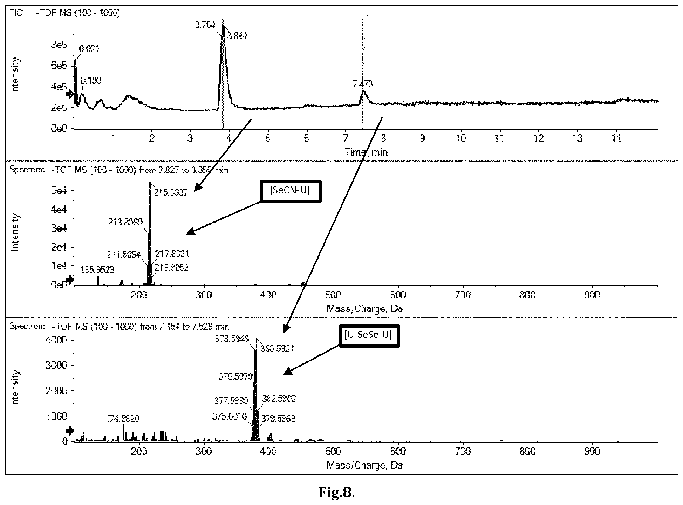

After purification the compound syntetized was used for the irradiation experiments. X-ray Cellrad chamber from Faxitron was used in this part. The products formed during the irradiation were analysed with the use of (1) analytical chromatograph Dionex Ultimate 3000 (Thermo Scientific), the column Wakopak Handy ODS 4,6x150 mm (Fig.7) and (2) LC-MS. LC analysis conditions: chromatograph Nexera X2 UHPLC, Shimadzu equipped in Kinetex 2.6 µm, C18, 100 Å, 2.1x150 mm column. The mobile phase consisted of solvents A" i B", in a 15-minutes gradient 0-15% B", where: A" - 0,2 % HCOOH; B" - 80% CH₃CN + 0.2 % HCOOH. The volumetric flow of effluents was equal to 0.3 mL/min. The detection was set to 254 nm wavelength.
MS: TripleTOF 5600, SCIEX. MS: CUR: 25; GS1: 30; GS2: 30; ISIF: 4500(-); TEM: 300; CE: 10.00; DP: 100. (Fig.8).

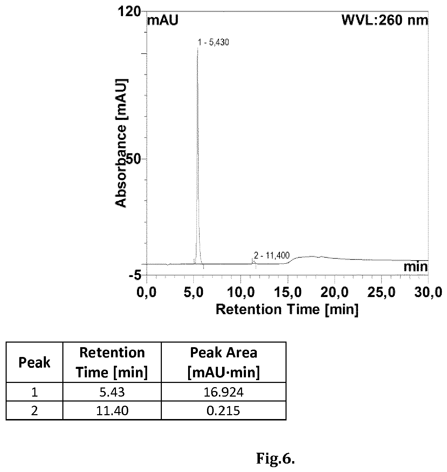

The compound tested was then dissolved in water with *tert*-butanol (0.03 M) in order to get a solution of 10⁻⁴ M concentration. The samples were deoxygenated by purging with argon in quartz capillaries 3x3x50 mm. The volume of the irradiated sample was 100 µL. The dose rate was equal to 140 Gy, with the source power of 3.5 Gy/min. (Fig.6, Fig.7, Fig.8).

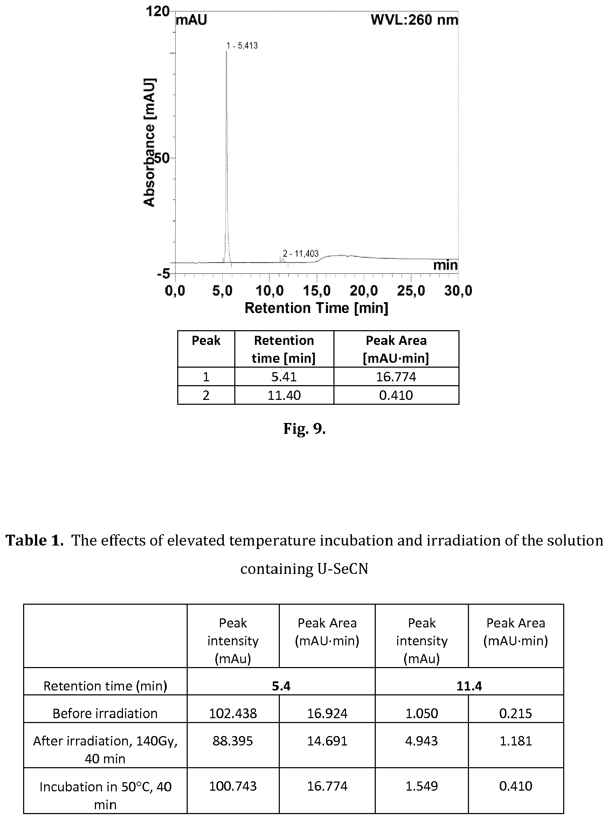

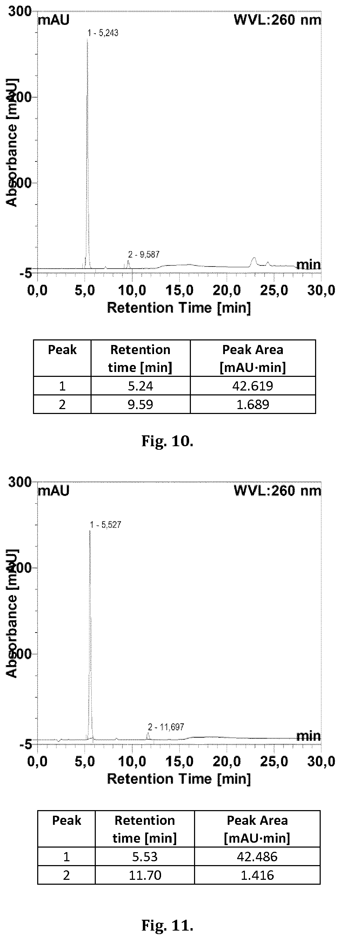

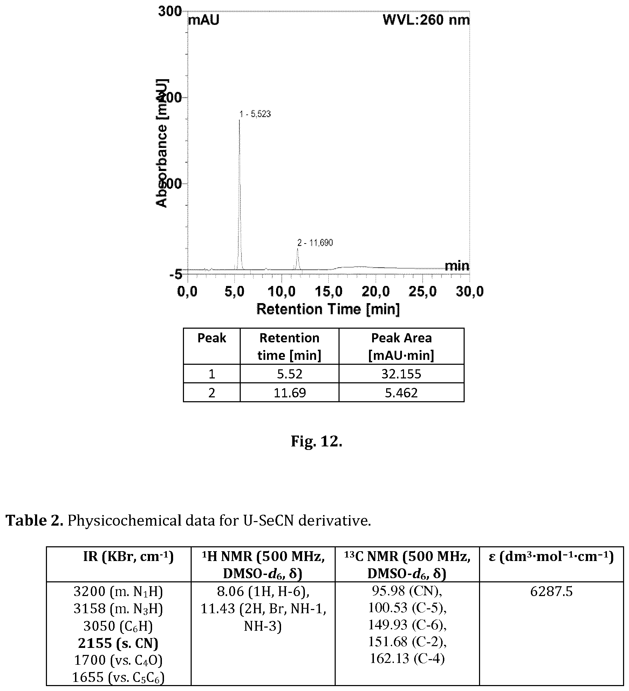

The results obtained in the radiolytic studies prove that the dose of ionization radiation used resulted in about 13 % decay (see Tab.1) of derivative (compare the signal intensities of retention time 5.4 min in control (Fig.6) and the irradiated sample (Fig.7). The irradiation was also accompanied with the increasing of peak intensity for retention time 11.4 min, by a factor of 4.7 (see Table 1). This peak corresponds to the radiolysis product - dimer U-SeSe-U, which mass was confirmed with the LC-MS analysis (Fig 8). The peak observed at 11.4 min of retention time is also present in the control sample, which suggests a slow hydrolysis of the synthesized derivative. To verify the stability of the compound, the solution containing U-SeCN was also incubated at the elevated temperature in 0.03 M tert-butanol, 50°C, for 40 min. The chromatograph of the solution after incubation is shown at Fig.9, and Table 1 shows the values of integral intensity of a peak of Rt 11.4 min before and after incubation. A small difference in the area under the peak 0.215 vs 0.410 suggests the slow hydrolysis of a derivative. Additionally, the subject of U-SeCN stability in water solution was investigated depending on the pH of solution. For this purpose, the U-SeCN derivative was dissolved to the concentration of 10⁻³ M in: (1) 0.1 % formic acid solution of pH 3; (2) 100 mM ammonium acetate solution of pH 5. (3) 100 mM Tris buffer of pH 7. The samples were then incubated in room temperature for 17 hours and the results (Fig. 10, Fig.11) show that the samples incubated in low pH (pH 3 i 5) were stable in these conditions. Only for the samples in pH 7 solution, the peak area for the Rt 11.7 min grows , with the simultaneously diminished area under Rt 5.5 min peak, that suggests, that increasing pH (to pH 7) could make the hydrolysis a bit easier for U-SeCN derivative and favor the dimer U-SeSe-U formation (Fig. 12).

## Claims

1. The method of synthesis of 5-Selenocyanatouracil) of the formula R1: 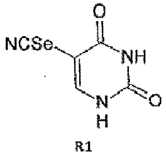 **characterized in that**:
- in anhydrous conditions, the compound R2: 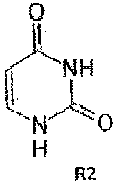 reacts with compound R4: ClSeCN, which is the donor of selenocyanato (SeCN) group,
- the compound R2 reacts with compound R4: ClSeCN which has been obtained by the reaction of the compound R3: KSeCN with chlorine gas.

2. The method of synthesis, according to claim 1, **characterized in that** the compound R2 reacts with the compound R4 in a molar ratio of 1:9.

3. The method of synthesis, according to claim 1, **characterized in that** the method is **characterized by** separate preparation of the compound R4 and subsequent addition of the compound R2 to it.

## Patentansprüche

1. Verfahren zur chemischen Synthese 5-Selenocynate-Uracil mit Formel R1: 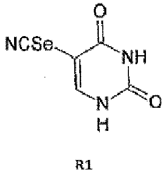 ist **dadurch gekennzeichnet, dass**:
- Molekül R2: 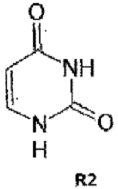 in wasserlosen Umständen reagiert mit dem Molekül R4: ClSeCN, das ein Elektronendonator der Selenocynat -Gruppe (SeCN) ist,
- das Molekül R2 reagiert mit dem Molekül R4: ClSeCN, das in der Reaktion vom Molekül R3: KSeCN mit Chlor in Gasform gewonnen wird.

2. Verfahren nach Anspruch 1 **gekennzeichnet durch**, Reaktion des Moleküls R2 mit dem Molekül R4 im Molverhältnis 1:9 gekennzeichnet.

3. Verfahren nach Anspruch 1 **gekennzeichnet durch**, separate Vorbereitung des Moleküls R4 und anschießend durch Additionsreaktion mit dem Molekül R2 gekennzeichnet.

## Revendications

1. Procédé de synthèse de 5-sélenocyanatouracile dont la formule R1: 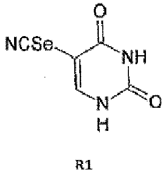 **caractérisée en ce que**:
- dans des conditions anhydres, la molécule R2: 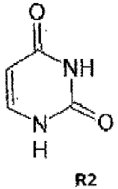 réagit avec la molécule R4: ClSeCN qui est donneur du groupe sélénocyanate (SeCN),
- la molécule R2 réagit avec la molécule R4: ClSeCN obtenue dans la réaction de la molécule R3: KSeCN avec chlore sous forme gazeuse.

2. Procédé selon la revendication 1,, est **caractérisée par** la réaction de la molécule R2 avec la molécule R4 dans un rapport molaire de 1:9.

3. Procédé selon la revendication 1, est **caractérisée par** la préparation séparée de la molécule R4, et ensuite par l'addition de la molécule R2 à celle'-ci.
